(19) Europäisches Patentamt
European Patent Office
Office européen des brevets

(11) **EP 2 438 428 B1**

(12) **FASCICULE DE BREVET EUROPEEN**

(45) Date de publication et mention de la délivrance du brevet:
**18.08.2021 Bulletin 2021/33**

(21) Numéro de dépôt: **10734785.8**

(22) Date de dépôt: **02.06.2010**

(51) Int Cl.:
*G01N 21/64* *(2006.01)*    *G02B 3/00* *(2006.01)*

(86) Numéro de dépôt international:
**PCT/FR2010/051076**

(87) Numéro de publication internationale:
**WO 2010/139900 (09.12.2010 Gazette 2010/49)**

(54) **SYSTÈME D'IMAGERIE À MICROLENTILLES ET DISPOSITIF ASSOCIÉ POUR LA DÉTECTION D'UN ÉCHANTILLON**

ABBILDENDE SYSTEM MIT MICROLINSEN FÜR PROBENMESSUNG

IMAGING SYSTEM WITH MICROLENSES AND ASSOCIATED DEVICE FOR THE DETECTION OF SAMPLES

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **02.06.2009 FR 0953631**

(43) Date de publication de la demande:
**11.04.2012 Bulletin 2012/15**

(73) Titulaire: **Commissariat à l'Énergie Atomique et aux Énergies Alternatives 75015 Paris (FR)**

(72) Inventeurs:
- **HAGUET, Vincent**
  **F-38100 Grenoble (FR)**
- **GABRIEL, Marion**
  **F-38000 Grenoble (FR)**
- **CHATELAIN, François**
  **F-38470 Beaulieu (FR)**
- **PICOLLETD'HAHAN, Nathalie**
  **F-35880 La Ferriere (FR)**

(74) Mandataire: **Lavoix 2, place d'Estienne d'Orves 75441 Paris Cedex 09 (FR)**

(56) Documents cités:
| | |
|---|---|
| EP-A2- 1 143 016 | WO-A1-01/03833 |
| WO-A1-2009/056065 | FR-A1- 2 812 943 |
| US-A1- 2001 021 534 | US-A1- 2002 182 111 |
| US-A1- 2005 046 847 | US-A1- 2005 237 524 |
| US-A1- 2008 290 383 | |

- **Chumin Zhao ET AL: "Large area CMOS active pixel sensor x-ray imager for digital breast tomosynthesis: Analysis, modeling, and characterization", Medical Physics, 1 November 2008 (2008-11-01), pages 6294-6308, XP055561856, United States DOI: 10.1118/1.4932368 Retrieved from the Internet: URL:http://ericfossum.com/Presentations/20 08%20Jan%20CMOS%20Image%20Sensors%20 Past%2 0Present%20and%20Future.pdf [retrieved on 2019-02-26]**

**EP 2 438 428 B1**

**Description**

La présente invention concerne un système d'imagerie à microlentilles et un dispositif associé destinés à la détection et à la caractérisation optique d'un échantillon.

L'invention s'applique dans le domaine de la miniaturisation d'instruments de mesure pour l'analyse chimique ou biologique.

En biologie, l'observation de cellules nécessite généralement l'utilisation d'un microscope optique afin de grossir l'image des cellules et de séparer les détails de cette image. Un équipement similaire est utilisé pour visualiser d'autres types d'objets biologiques, tels que des bactéries, des levures, des champignons, des pollens, des algues, des liposomes, ainsi que des particules d'origine synthétique. Pour cela, un microscope comporte des lentilles optiques (objectif, oculaire), une source lumineuse et au moins un photocapteur. Les microscopes utilisés quotidiennement par les chercheurs sont souvent coûteux et contraignants, notamment à cause de leur encombrement spatial. Généralement, ils ne permettent pas de produire des images de manière continue dans le temps ou à des moments prédéfinis.

L'une des voies de miniaturisation envisagées actuellement est de supprimer les objectifs de grossissement du microscope et de réaliser l'image de l'ombre ou la figure de diffraction créée sur la surface sensible des photocapteurs. L'image formée par l'ombre ou la figure de diffraction de l'objet observé au contact des photocapteurs est appelée photogramme. A priori la résolution, définie comme la capacité de distinguer deux disques très proches, est dans ce type de système limitée par la taille des pixels et la distance objet/surface du capteur optique. Alors que dans un microscope classique, elle est limitée par la diffraction dans le système de lentilles des objectifs.

Il existe des systèmes décrivant un système d'imagerie d'un échantillon comprenant une matrice de photocapteurs, une première lamelle disposée en regard de la matrice de photocapteurs définissant une face de support de l'échantillon, et un ensemble d'éléments optiques, disposé entre la matrice de photocapteurs et la première lamelle, l'ensemble d'éléments optiques comportant une matrice de microlentilles, une microlentille étant située au-dessus de chaque photocapteur de la matrice de photocapteurs.

Cependant les images acquises par un tel système d'imagerie ne montrent ni la morphologie ni la forme dans le cas où les cellules de l'échantillon à analyser sont adhérentes. En effet, les cellules en suspension ou adhérées ne correspondent pas au même défi : les cellules sont des objets de faible contraste avec le milieu de culture car les indices optiques sont similaires. Lorsqu'elles sont adhérées, elles présentent une forme plate et allongée, ce qui les rend plus difficiles à imager.

Il est également connu un système d'imagerie du document US 2001/021534 A1.

Le but de l'invention est de proposer un dispositif de taille réduite apte à imager des cellules, en particulier des cellules adhérentes, pour localiser et identifier les cellules, les dénombrer et analyser leur morphologie. La taille réduite du dispositif permet de l'introduire directement dans un incubateur afin de permettre la prolifération des cellules de l'échantillon.

A cet effet, l'invention a pour objet un système d'imagerie d'un échantillon comprenant les caractéristiques de la revendication 1.

Suivant des modes particuliers de réalisation, le système d'imagerie d'un échantillon comporte l'une ou plusieurs des caractéristiques des revendications 2 à 15.

Ainsi, ce système d'imagerie de taille réduite est apte à produire des images de cellules de manière continue dans le temps ou à des temps prédéfinis pour quantifier la prolifération des cellules, suivre individuellement leur déplacement et en déduire leur trajectoire et leur vitesse, détecter les divisions des cellules et établir des filiations, mettre en évidence et quantifier des changements de morphologie, identifier des événements cellulaires d'intérêt (événements rares...).

L'invention a également pour objet un dispositif de détection et de caractérisation d'un échantillon selon la revendication 16.

Suivant des modes particuliers de réalisation, le dispositif de détection et de caractérisation d'un échantillon comporte l'une ou plusieurs des caractéristiques des revendications 17 à 19.

L'invention sera mieux comprise à la lecture de la description qui va suivre, donnée uniquement à titre d'exemple, et faite en se référant aux dessins annexés, sur lesquels :

- la figure 1 est une vue schématique d'un dispositif de détection et de caractérisation d'un échantillon selon l'invention,
- la figure 2 est une vue schématique en coupe d'un système optique d'un système d'imagerie adapté à la détection et à la caractérisation d'un échantillon selon l'invention,
- les figures 3 et 5 sont des vues identiques à celle de la figure 2 de modes de réalisation différents d'un système optique d'un système d'imagerie selon l'invention,
- la figure 4 est une vue identique à la figure 2 d'un mode de réalisation d'un système optique d'un système d'imagerie non revendiqué,
- la figure 6 est une vue schématique d'un autre mode de réalisation d'un système d'imagerie selon l'invention,
- les figures 7 et 8 sont des vues schématiques d'une lamelle intégrée dans un système optique d'un système d'imagerie et comportant un système microfluidique selon l'invention, et
- les figures 9, 10 et 11 sont des vues schématiques de trois autres modes de réalisation d'un dispositif de détection et de caractérisation d'un ou de plusieurs échantillons selon l'invention.

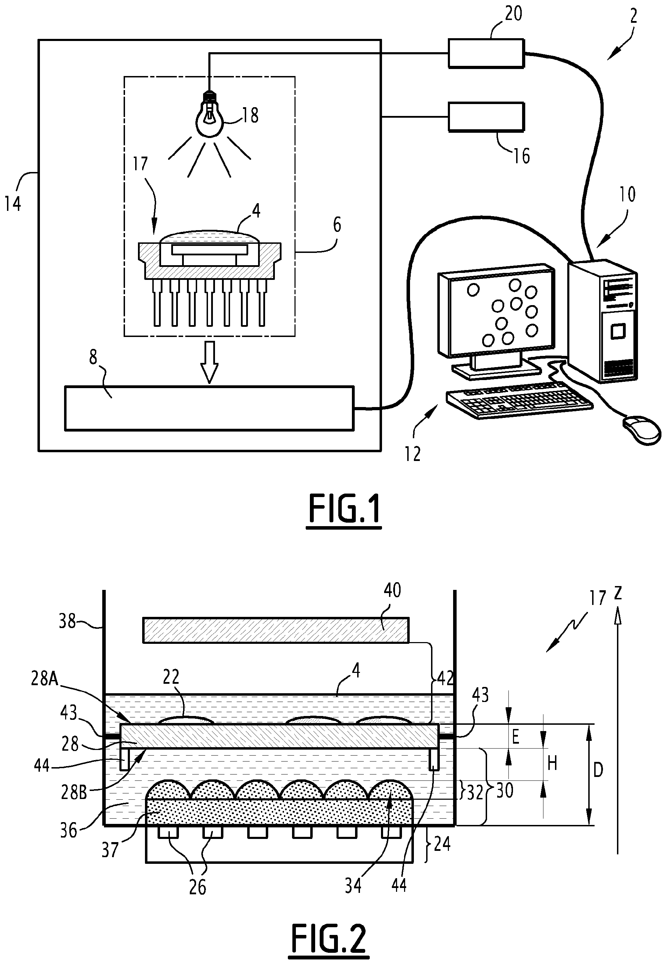

L'invention concerne un dispositif 2 de détection et de caractérisation d'un échantillon 4, dont un mode de réalisation est illustré sur la figure 1. L'échantillon 4 comprend des objets 5 à détecter et imager afin d'étudier leurs caractéristiques (morphologie, taille...). Les objets 5 sont des objets de taille micrométrique (bactéries, levures, champignons, pollens, algues, liposomes...) et de préférence des cellules.

Le dispositif 2 comprend au moins un système d'imagerie 6 destiné à détecter et à caractériser l'échantillon 4 et un contrôleur électronique 8 apte à contrôler le système d'imagerie 6. Le contrôleur électronique 8 comprend un support apte à fixer le système d'imagerie 6, par exemple pour l'enficher si celui-ci comprend des fiches de connexion.

Le système d'imagerie 6 est décrit en détail dans la suite.

Le dispositif 2 comporte en outre un système informatique 10 destiné à piloter le contrôleur électronique 8 auquel il est relié.

Le système informatique 10 comprend une interface homme/machine 12 destinée à entrer des informations pour piloter le contrôleur électronique 8 et à afficher des informations concernant l'échantillon 4.

Le système informatique 10 comporte en outre une unité de calcul apte à réaliser des traitements sur les images et une unité de stockage pour enregistrer les images acquises de l'échantillon.

Le dispositif 2 comprend une chambre d'incubation ou incubateur 14 et des moyens 16 de contrôle et de régulation du taux de $CO_2$, de l'humidité et de la température à l'intérieur de la chambre d'incubation. Par exemple, l'incubateur 14 est un incubateur standard pour la biologie cellulaire, de dimensions de l'ordre du mètre cube de façon connue, pouvant éventuellement contenir plusieurs systèmes d'imagerie 6, ou un incubateur miniaturisé aux dimensions d'un système d'imagerie 6. Dans ce dernier cas, le dispositif 2 est facilement transportable pour être utilisé par exemple, à proximité d'un lieu de collecte de l'échantillon.

Le système d'imagerie 6, le contrôleur électronique 8 et l'échantillon 4 sont disposés à l'intérieur de la chambre d'incubation 14.

Le système d'imagerie 6 comporte un système optique 17 destiné à analyser l'échantillon 4 et, de préférence, le système d'imagerie 6 comporte au moins une source lumineuse 18 destinée à éclairer l'échantillon 4, celui-ci étant disposé entre la source lumineuse 18 et le système optique 17.

La source lumineuse 18 est située en regard du système optique 17 afin de réaliser une image en transmission de l'échantillon et à une distance contrôlée, par exemple à une distance de quelques centimètres. La source lumineuse 18 est située à la verticale du système optique 17.

Selon une variante, la source lumineuse 18 est décalée par rapport à la verticale du système optique 17, par exemple à une distance de quelques centimètres selon un arc de cercle autour du système optique 17.

En outre, la source lumineuse est non ou faiblement collimatée avec un angle limite d'éclairage inférieur à 20°, par exemple 10°.

Néanmoins, selon les besoins de l'opérateur et de façon connue, la source lumineuse peut être collimatée par un système de lentilles, diaphragmes et/ou filtres directionnels ou non, polychromatique ou monochromatique, polarisée ou non, étendue ou ponctuelle.

La source lumineuse 18 est apte à éclairer avec une longueur d'onde comprise entre 200 nm et 800 nm, c'est-à-dire émettant dans le visible et/ou le proche ultraviolet (UV). De préférence, c'est une lumière blanche, c'est-à-dire une lumière émettant un spectre continu de longueur d'onde, en particulier dans le domaine visible (400 nm à 800 nm).

Par exemple, la source lumineuse 18 comprend une diode électroluminescente (DEL ou LED en anglais), par exemple, une DEL blanche, un réseau de DELs, une ampoule à incandescence, ou une feuille électroluminescente ou une LED plate telle qu'une XLamp commercialisée par Cree (Durham, Etats-Unis).

Selon une variante, la source lumineuse 18 comporte un dispositif d'illumination de type Kölher, c'est-à-dire un système d'imagerie couramment utilisé dans les microscopes optiques pour l'illumination de l'objet ou échantillon.

Des moyens 20 de commande de la source lumineuse 18 sont intégrés au dispositif 2 et connectés au système informatique 10.

Selon une variante, la source lumineuse 18 est positionnée au-dessus du système optique 17 de façon à ce qu'elle éclaire l'échantillon selon un angle spécifique (angle d'éclairage) contrôlé par l'opérateur ou par le système informatique 10.

La source lumineuse 18 éclaire l'objet ou échantillon à une intensité et/ou pendant des durées et/ou à des instants spécifiés par l'opérateur ou par le système informatique 10.

Selon les besoins de l'opérateur, la source lumineuse 18 peut être commune à plusieurs échantillons ou n'éclairer qu'un seul échantillon.

Le fonctionnement du dispositif 2 de détection et de caractérisation d'un échantillon 4 selon l'invention va maintenant être décrit.

Afin d'étudier et d'analyser l'échantillon 4, un opérateur le dispose dans le système d'imagerie 6. Le système d'imagerie 6 est connecté au contrôleur électronique 8, par exemple en enfichant le système optique 17 sur le support du contrôleur électronique 8.

Si nécessaire, l'ensemble comprenant le système optique 17, l'échantillon 4, le contrôleur électronique 8 et la source lumineuse 18, est placé dans la chambre d'incubation 14, en particulier pour permettre la prolifération normale de la population cellulaire. L'incubateur 14 régule la température et le taux de $CO_2$, et maintient

un niveau d'humidité élevé grâce aux moyens 16 de contrôle et de régulation préalablement réglés par l'opérateur.

En outre, cet ensemble (système d'imagerie 6 dont la source lumineuse 18, échantillon 4 et contrôleur électronique 8) est de préférence placé dans une chambre noire afin de réduire les bruits parasites liés à l'éclairage ambiant.

Ensuite l'opérateur entre des informations par l'interface homme/machine 12 pour régler la ou chaque source lumineuse 18 via les moyens 20 de commande de l'éclairage et pour piloter le système optique 17 du système d'imagerie 6 par l'intermédiaire du contrôleur électronique 8. L'opérateur contrôle ainsi à distance, grâce au système informatique 10, le ou chaque système d'imagerie 6, dont la ou chaque source lumineuse 18.

En fonctionnement, la ou chaque source lumineuse 18 éclaire l'échantillon 4. La lumière ou signal optique (photons) transmis par l'échantillon 4 est transformé par le système optique 17 du système d'imagerie 6 en signal électrique. Le signal électrique est ensuite transmis par le contrôleur électronique 8 au système informatique 10.

Les données acquises par le système optique 17 du système d'imagerie 6 sont enregistrées dans l'unité de stockage du système informatique 10. Puis, l'unité de calcul reconstruit l'image ou séquence d'images des cellules 5 de l'échantillon 4 à partir des données. En effet, le dispositif enregistre de façon connue, des événements dynamiques, soit en réalisant une acquisition en continu (mode vidéo) pour obtenir un film, soit en acquérant des images à intervalle régulier (mode photo) pour obtenir une séquence d'images, et pas uniquement l'intensité du signal optique détectée en transmission de l'échantillon.

Enfin, l'opérateur commande, par l'interface homme/machine 12, l'unité de calcul pour réaliser d'autres traitements de l'image ou séquence d'images afin de caractériser l'échantillon 4. Par exemple, l'unité de calcul peut repérer spatialement des cellules 5 de l'échantillon, identifier les cellules 5 sur la base d'une analyse de leur morphologie et/ou de signaux fluorescents et/ou luminescents spécifiques, dénombrer les cellules, comparer leur position avec leur position antérieure, enregistrer la trajectoire et la vitesse de migration de chaque cellule, quantifier la prolifération des cellules, caractériser la morphologie de chaque cellule (calcul du périmètre et de l'aire couverte par chaque cellule, critère de rotondité de chaque cellule, texture du contour), mettre en évidence des changements morphologiques sur des cellules individuelles ou d'une population de cellules, segmenter l'image pour tracer les jonctions entre les cellules et calculer le nombre de cellules voisines, détecter des événements d'intérêt tels que des divisions cellulaires ou des différentiations ou des bouffées (ou « *bursts* » en anglais) calciques, établir des filiations, et/ou localiser des organelles (noyaux,...) dans les cellules. En outre, elle peut éliminer le bruit de fond, par exemple en réalisant pour chaque expérience une image en fond clair (lumière parasite) et en fond sombre (courant d'obscurité). Elle peut également reconstituer des films de la culture cellulaire sur l'ensemble ou une partie de l'image.

Selon un autre mode de réalisation pour un échantillon 4 émettant de la lumière, par exemple une bioluminescence ou une chimiluminescence, le dispositif 2 est utilisé sans aucune source lumineuse 18. Dans ce cas, les analyses nécessitent d'être effectuées dans le noir total (en chambre noire). L'intensité de la luminescence est intégrée pendant une durée déterminée par l'opérateur afin d'augmenter la sensibilité de la mesure, par exemple le temps d'intégration de la luminescence est de 5 min.

La figure 2 est une vue schématique en coupe du système optique 17 du système d'imagerie 6 destiné à détecter et à caractériser l'échantillon 4. Par exemple, l'échantillon 4 comprend des objets, par exemple des cellules 5 et de préférence des cellules biologiques adhérées 22 dans un milieu aqueux, de façon connue un milieu de culture.

Le système optique 17 du système d'imagerie 6 comprend une matrice 24 de photocapteurs 26, régulièrement arrangés, dont la zone photosensible de chaque photocapteur 26 est par exemple en silicium.

Le système optique 17 comporte en outre une première lamelle transparente 28 disposée en regard de la matrice 24 de photocapteurs 26 définissant une face 28A de support de l'échantillon 4. La face 28A est de préférence plate et transparente.

De préférence, la première lamelle 28 a un traitement de surface spécifique pour capturer et/ou favoriser l'adhésion des cellules 22 sur la face 28A de support de l'échantillon 4, de façon connue par un procédé de fonctionnalisation avec des protéines appropriées, de préférence des protéines de la matrice extracellulaire par exemple la fibronectine, ou avec un plasma oxygène.

En outre, un autre traitement de surface évite l'adhésion sur la face 28B opposée à la face 28A, support de l'échantillon, par exemple en utilisant de la polylysine-PEG (polylysine-polyéthylène glycol) qui lui confère des propriétés d'hydrophobie.

Par exemple, la face 28A de support des cellules adhérées 22 a un traitement de surface pour augmenter l'adhésion des cellules sur celle-ci et l'autre face 28B a un traitement de surface pour réduire ou supprimer l'adhésion des cellules 22.

Par exemple, la première lamelle 28 sert de support à la culture cellulaire.

La première lamelle 28 a de bonnes qualités en termes de transmission optique, par exemple elle est en verre ou en polystyrène.

Le système optique 17 du système d'imagerie 6 comprend en outre un ensemble 30 d'éléments optiques disposé entre la matrice 24 de photocapteurs 26 et la première lamelle 28.

L'ensemble 30 d'éléments optiques comporte un élément séparateur 37 situé en regard et en contact de la matrice 24 de photocapteurs, et une matrice 32 de microlentilles 34 disposée entre l'élément séparateur 37 et la première lamelle 28.

La matrice 32 de microlentilles 34 est positionnée au-dessus de la matrice 24 de photocapteurs 26 de façon telle qu'une microlentille est située au-dessus de chaque photocapteur 26 de la matrice 24 de photocapteurs. Ainsi, chaque microlentille 34 est associée à un photocapteur 26. L'axe optique de chaque microlentille 34 est sensiblement confondu avec l'axe optique d'un photocapteur 26.

Par exemple, l'axe optique d'une microlentille 34 et l'axe optique d'un photocapteur 26 peuvent être légèrement décalés, le décalage (angulaire ou en distance) étant inférieur ou égale à 20 %. Ce décalage est soit involontaire en résultant d'une difficulté à aligner la matrice de microlentilles avec la matrice de photocapteurs, soit volontaire afin de réorienter un faisceau optique incident oblique vers le photocapteur grâce à la microlentille.

L'élément séparateur 37 est formé d'un ou plusieurs matériaux optiquement transparents, par exemple, de filtres ou de couches de passivation.

L'élément séparateur a pour fonction d'éloigner la matrice 24 de photocapteurs 26 de la matrice 32 de microlentilles 34 afin que les rayons lumineux soient concentrés ou focalisés vers les photocapteurs.

L'ensemble 30 d'éléments optiques comprend en outre un premier milieu 36, d'indice de réfraction ou indice optique n, disposé entre la matrice 32 de microlentilles et la première lamelle 28. Le premier milieu optique 36 est destiné à modifier la focale de chaque microlentille 34. En effet, dans le premier milieu 36, d'indice optique n, la focale de chaque microlentille 34 est

$$F_n = \frac{n_{lentille} - n_{air}}{n_{lentille} - n} \times F ,$$

où F est la focale de chaque microlentille 34 mesurée dans l'air.

L'indice de réfraction du premier milieu optique 36 est sensiblement compris entre 1 et l'indice de réfraction des microlentilles 34. De préférence, le milieu 36 est un gel ou un liquide, par exemple une huile.

Le système optique 17 du système d'imagerie 6 comporte en outre une seconde lamelle 40 disposée telle que les cellules 22 sont situées entre la première lamelle 28 et la seconde lamelle 40 dans un second milieu 42 délimité par les deux lamelles 28, 40.

Avantageusement, la seconde lamelle 40 a un traitement de surface parmi ceux déjà décrits pour la première lamelle 28.

Le second milieu 42 comprend un liquide, par exemple un liquide biologique apte à la culture cellulaire tel que classiquement du DMEM (pour « *Dulbecco's Modified Eagle's Medium* » en anglais) ou une solution saline telle qu'un tampon phosphate salin ou PBS (pour « *Phosphate Buffered Saline* » en anglais).

Le niveau du liquide du second milieu est tel qu'il recouvre la face 28A de support de l'échantillon 4 et les objets 22.

Selon une variante, le premier milieu 36 est un liquide similaire à celui du second milieu 42.

Le système optique 17 du système d'imagerie 6 comprend une cuvette 38 étanche dans laquelle sont placés la matrice 30 d'éléments optiques, la première lamelle 28, l'échantillon 4 de sorte à contenir les liquides ou gels du premier milieu 36 et/ou du second milieu 42.

Le fond de la cuvette 38 peut coïncider avec la surface des photocapteurs 26. Par exemple, la cuvette 38 est percée au fond et collée avec la matrice 24 de photocapteurs 26 afin de garantir l'étanchéité grâce à une colle biocompatible.

Le système optique 17 du système d'imagerie 6 comporte en outre des moyens 43 de séparation aptes à séparer le premier milieu 36 du second milieu 42 si ceux-ci sont différents. Ces moyens de séparation 43 sont fixés aux parois de la cuvette 38 ou à la première lamelle 28.

Selon une autre variante, la distance mesurée suivant l'axe optique Z entre la première lamelle 28 et la seconde lamelle 40 est réglée afin d'obtenir de façon connue une configuration dite entre lame et lamelle de l'échantillon 4. La seconde lamelle 40 est dans cette configuration en contact avec l'échantillon 4.

La seconde lamelle 40 peut favoriser des échanges gazeux à travers la seconde lamelle 40, et/ou empêcher une évaporation à travers la seconde lamelle 40 d'une partie du second milieu 42 par exemple du milieu de culture et de l'échantillon 4.

Le système optique 17 comprend en outre des moyens de réglage 44 des distances mesurées suivant l'axe optique Z entre la face de support 28A de l'échantillon de la première lamelle 28 et la face des photocapteurs 26 disposée en regard de la première lamelle 28.

De préférence, ces moyens de réglage 44 comportent au moins une céramique piézoélectrique. Les moyens de réglage 44 peuvent être fixés mécaniquement au fond ou sur les bords de la cuvette 38 ou sur le support de la matrice 24 de photocapteurs 26.

Selon une autre variante, la première lamelle 28 est fixée à une hauteur fixe aux parois de la cuvette 38 du système d'imagerie 6 par un moyen mécanique, électrostatique, magnétique ou équivalent.

La première lamelle 28, et donc l'échantillon 4 et les objets 5, de préférence des cellules adhérées 22, peuvent être retirés du système optique 17 du système d'imagerie 6, en fonction des besoins de l'opérateur, par exemple pour une caractérisation de l'échantillon sur un autre instrument ou pour un traitement supplémentaire de l'échantillon (coloration, marquage avec un fluorophore,...). La première lamelle 28, et l'échantillon ou objet, peuvent de même être repositionnés dans le système d'imagerie 6.

Le fonctionnement du système d'imagerie 6 selon l'invention va maintenant être décrit.

Au cours du procédé d'analyse d'un échantillon par le dispositif 2 décrit ci-avant, le fabricant ou l'opérateur du système d'imagerie choisit préalablement l'élément séparateur 37 et la première lamelle 28 en fonction de leurs caractéristiques optiques (matériaux, indice de réfraction, épaisseur...) et de l'utilisation/application désirée.

Ensuite, la matrice 32 de microlentilles 34 est choisie en tenant compte de l'angle du cône d'acceptance des photocapteurs 26 qui est contrôlé par la présence des microlentilles 34, en particulier par la nature (indice de réfraction) et la forme (rayon de courbure) de chaque microlentille 34.

Chaque microlentille 34 comprend un dioptre plan et un dioptre convexe. La face supérieure en regard de l'échantillon des microlentilles est le dioptre convexe assimilable à une calotte sphérique. De préférence, les microlentilles sont fabriquées en polystyrène, en polyimide, en photorésine AZ4562 ou similaire, en poly(diméthylsiloxane), en SU-8, en oxyde de silicium, en verre silicate dopé au bore et au phosphore (BPSG), ou en un matériau thermoplastique transparent tel que le polyméthacrylate de méthyle (PMMA) ou le polycarbonate.

La focale de chaque microlentille 34 est inférieure à 25 $\mu$m, préférentiellement inférieure à 10$\mu$m, de façon à limiter les phénomènes de diaphotie optique (ou « optical cross-talk » en anglais) entre les microlentilles et les photocapteurs associés sous-jacents.

De préférence, le diamètre d'ouverture de chaque microlentille est au moins deux fois inférieur à la plus petite dimension latérale d'une cellule. La plus petite dimension latérale d'une cellule est la plus petite dimension de la cellule passant par son centre de gravité et mesurée dans un plan perpendiculaire à l'axe optique du système d'imagerie, c'est-à-dire dans un plan parallèle à la face 28A de la lamelle servant de support à la cellule.

En outre, la distance entre les axes optiques de deux microlentilles plus proches voisines est de préférence, inférieure à au moins 30 % du diamètre d'ouverture d'une microlentille. Pour rappel, le diamètre d'ouverture d'une microlentille est le diamètre de la section d'intersection du dioptre plan et du dioptre convexe formant la microlentille.

Les photocapteurs 26 associés aux microlentilles 34 sont agencés pour qu'un objet 5, de préférence une cellule, soit couvert par au moins deux photocapteurs adjacents dans une première direction et deux autres photocapteurs adjacents dans une seconde direction perpendiculaire à la première direction.

Ainsi l'image d'une cellule est réalisée par au moins une matrice de 2 x 2 photocapteurs adjacents.

Par exemple, le diamètre d'ouverture de chaque microlentille est sensiblement compris entre 0,7 et 10 $\mu$m, de préférence entre 0,7 $\mu$m et 3,0 $\mu$m, pour imager des objets tels que des cellules dont le diamètre est sensiblement compris entre 20 et 30 $\mu$m. Ces valeurs permettent d'éviter une discontinuité dans l'image des objets, quitte à ce qu'une même portion de la cellule contribue à l'intensité lumineuse de deux photocapteurs voisins. Un sur-échantillonnage de l'objet 5 est préférable à un sous-échantillonnage afin de produire des images de bonne qualité des cellules.

Chaque cellule est ainsi représentée sur l'image par au moins 2 pixels de côté, de préférence par au moins 6 pixels de côté, de manière à faciliter le traitement informatique postérieur sur les images des cellules : plus les cellules sont représentées par un grand nombre de pixels, plus les opérations d'amélioration de contraste, de filtrage et de seuillage d'intensité réalisées lors du traitement informatique des images seront conformes à la réalité.

Par exemple, la distance entre les axes optiques de deux microlentilles plus proches voisines est inférieure à 3,0 $\mu$m pour des microlentilles de 10 $\mu$m de diamètre d'ouverture, inférieure à 900 nm pour des microlentilles de 3,0 $\mu$m de diamètre d'ouverture, inférieure à 210 nm pour des microlentilles de 700 nm de diamètre d'ouverture.

Ensuite, le fabricant ou l'opérateur choisit le premier milieu 36 en fonction de son indice de réfraction n afin de modifier la focale des microlentilles 34 et ainsi l'angle d'acceptance des photocapteurs 26. En effet, les faisceaux lumineux sont déviés à l'interface optique entre le premier milieu optique 36 et chaque microlentille 34 selon la loi de Descartes. Ainsi, l'indice du premier milieu 36 permet également de régler l'angle du cône d'acceptance des photocapteurs.

Plus précisément, la sélection de l'indice de réfraction du premier milieu 36 contrôle la position des croisements des cônes d'acceptance de photocapteurs voisins ou adjacents. Ainsi, l'angle du cône d'acceptance des photocapteurs 26 est augmenté, et par conséquent la position des croisements des cônes d'acceptance de photocapteurs voisins est diminuée, en augmentant l'indice de réfraction n du milieu 36, et inversement.

Par exemple, pour une microlentille 34 en polystyrène d'indice de réfraction 1,61, il suffit de remplacer le milieu 36, initialement de l'air (soit n = 1,0003), par une huile d'indice de réfraction 1,55 pour élargir le cône d'acceptance des photocapteurs 26.

L'indice de réfraction du premier milieu 36 est sensiblement compris entre 1 et l'indice de réfraction des microlentilles 34, de préférence entre 1 et 1,64.

En fait, tout matériau optiquement actif, situé entre la matrice de photocapteurs et l'échantillon, participe à la modification du cône d'acceptance des photocapteurs. En particulier, c'est le cas de l'élément séparateur 37 et de la première lamelle 28. Pour cette dernière, les faisceaux lumineux sont déviés à l'interface optique entre la première lamelle 28 et le premier milieu optique 36 selon la loi de Descartes. Par conséquent le choix de la première lamelle ajuste également le cône d'acceptance des photocapteurs. Néanmoins, elle est également choisie pour ses qualités optiques (transmission élevée) et est utilisée comme support pour les objets 5 et de préférence les cellules adhérées 22 à imager.

Ensuite, grâce aux moyens de réglage 44, la distance, mesurée suivant l'axe optique Z, entre la face 28A de support de l'échantillon 4 de la première lamelle 28 et le sommet des microlentilles 34, est réglée, et par conséquent l'épaisseur du premier milieu 36 aussi. La distance est réglée afin d'être comprise entre 0 et 1500 μm, et de préférence entre 100 et 500 μm. La distance revendiquée est comprise entre 100 μm et 1500 μm.

De préférence, cette distance est inférieure ou égale à $-150 \times F \times n \times 400 \times F$, où F est la focale des microlentilles 34 mesurée dans l'air et n l'indice de réfraction du premier milieu optique 36.

On note H la distance mesurée suivant l'axe optique Z, entre la face de la première lamelle 28 en regard des microlentilles et le sommet des microlentilles. Le réglage de la distance H permet alors d'obtenir une image plus ou moins nette et/ou plus ou moins contrastée des cellules 5, de préférence des cellules adhérées 22 de l'échantillon 4 en fonction de ce que l'opérateur souhaite observer comme détails des cellules 5.

En effet, la variation de la distance D entre l'objet 5 et la surface des photocapteurs a pour effets :

- de modifier la largeur ou le grandissement de l'image de l'objet, c'est-à-dire l'étalement sur un nombre variable de photocapteurs adjacents : plus l'objet est éloigné de la matrice 24 de photocapteurs 26, plus il est imagé par un grand nombre de photocapteurs 26, et ainsi plus l'objet apparaît large sur l'image finale produite,
- de mettre en évidence le contour diffracté de l'objet 5, de préférence une cellule adhérée 22, à partir d'une certaine hauteur. Proche de la surface, le motif de diffraction sur le contour de l'objet n'est pas mis en évidence car le motif de diffraction est concentré sur un seul photocapteur, ce qui ne permet pas de révéler les détails spatiaux du motif de diffraction. Lorsqu'on s'éloigne progressivement de la surface, le motif de diffraction sur le contour de l'objet s'étale sur 2, 3, 4,... photocapteurs adjacents et est de mieux en mieux observé. Néanmoins, loin de la surface, le motif de diffraction est diffus sur un trop grand nombre de photocapteurs (il n'est alors pas possible d'établir une image exploitable de l'objet si l'image d'un point de l'objet s'étale sur plus de 200 photocapteurs), et
- de modifier le contraste du contour de l'objet.

Les propriétés optiques des faces 28A et 28B de la première lamelle 28 peuvent être optimisées pour renforcer les effets de diffraction, de diffusion, d'interférences et de dispersion permettant de visualiser le contour des cellules 22.

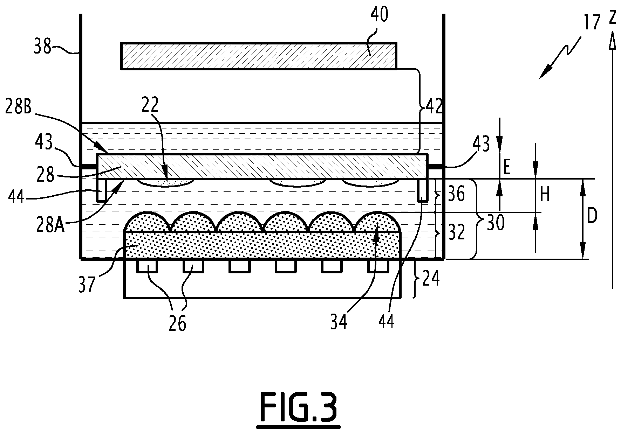

Dans un deuxième mode de réalisation du système optique 17 du système d'imagerie 6 illustré sur la figure 3, la face 28A de la première lamelle est située en regard de la matrice 24 de photocapteurs 26 ; l'échantillon 4 comprenant des cellules 5, de préférence adhérées 22 à la face 28A de la première lamelle, est donc disposé entre la première lamelle 28 et la matrice 32 de microlentilles 34.

Ce mode de réalisation permet de s'affranchir de l'épaisseur de la première lamelle 28 dans le réglage de la distance entre les objets à imager et le sommet des microlentilles. Cette configuration a l'avantage d'obtenir des distances très petites, quasi-nulles, entre les objets 22 et les microlentilles 34, et de réaliser des images au plus près des photocapteurs 26.

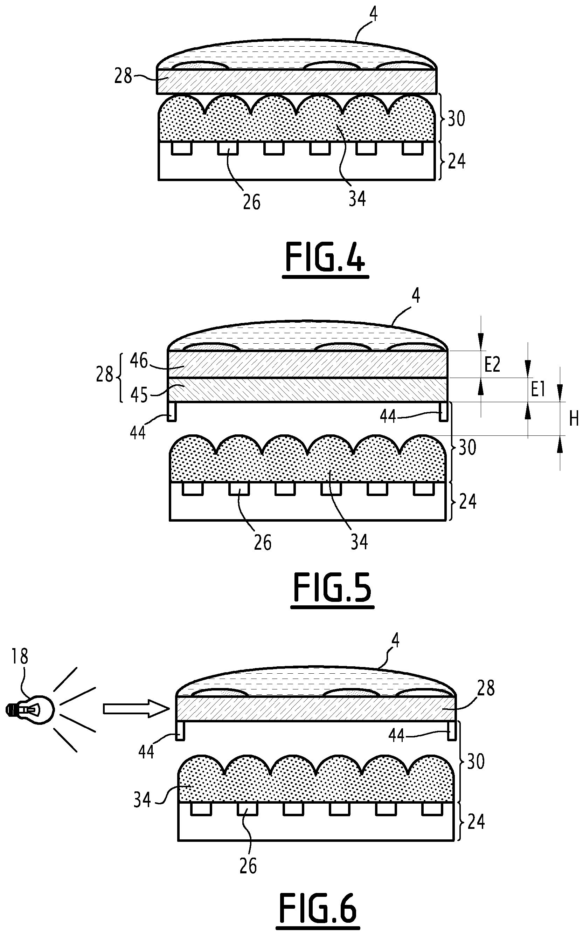

Selon un troisième mode de réalisation représenté sur la figure 4 et non revendiqué, la première lamelle 28 est posée sur le sommet des microlentilles 34. La distance H entre le sommet des microlentilles et la face 28B en regard des microlentilles de la première lamelle 28 est alors nulle.

Dans ce cas, le réglage de la distance entre la face 28A de support de l'échantillon et le sommet des microlentilles, revient à choisir une épaisseur E de la première lamelle 28, adaptée pour que la distance mesurée, selon l'axe optique Z des photocapteurs 26, entre la face de support de l'échantillon 28A et le sommet des microlentilles 34, soit sensiblement comprise entre 0 et 1500 μm, et de préférence entre 100 et 500 μm.

Néanmoins, il faut noter que le premier milieu 36 est toujours présent dans les interstices entre les microlentilles 34 et la première lamelle 28, et par conséquent agit toujours sur les cônes d'acceptance des photocapteurs 26.

Selon une quatrième variante représentée sur la figure 5, la première lamelle 28 comporte une lame 45, d'épaisseur E1, et une lamelle amovible 46, d'épaisseur E2, accolée à la lame 45 et définissant la face de support de l'échantillon ; la somme des épaisseurs E1 et E2 est par conséquent égale à l'épaisseur E de la première lamelle 28. Cette variante facilite également le retrait de l'échantillon sans modifier la distance H entre la première lamelle 28 et le sommet des microlentilles 34 préalablement réglée.

La présence de la seconde lamelle 40, par exemple en verre, est surtout nécessaire dans le cas d'une utilisation en fluorescence, par exemple pour imager des fluorophores ou des « quantum dots ». Dans ce mode de réalisation, la première et la seconde lamelles 28, 40 sont de préférence des filtres optiques.

De manière préférentielle pour cette utilisation, la source lumineuse 18, alors source d'excitation, est monochromatique.

Le second filtre 40 n'est pas nécessaire si la longueur d'onde d'excitation est filtrée par le premier filtre 28. Par exemple en supprimant la seconde lamelle 40 et en utilisant une première lamelle 28 de verre comme filtre, l'absorbance du verre dans l'ultra-violet (UV) filtre la lumière d'excitation si elle est dans l'UV. Un deuxième exemple consiste en une première lamelle 28 dont la face 28A ou la face 28B est recouverte d'un empilement filtrant, réalisé par empilement de couches diélectriques, qui absorbe la longueur d'onde d'excitation.

Selon une variante, le système optique 17 du système d'imagerie 6 comprend un filtre, ou un réseau de filtres dédiés, situé entre la matrice 24 de photocapteurs 26 et la matrice 32 de microlentilles 34. Par exemple, ce filtre, ou réseau de filtres, est intégré dans l'élément séparateur 37. Les filtres peuvent être différents sous des microlentilles adjacentes afin de caractériser un même échantillon 4 selon au moins deux longueurs d'onde différentes.

Selon une autre variante, la seconde lamelle 40 est un filtre directionnel afin de compenser l'absence d'un système de collimation de la source lumineuse 18 qui entraîne des distorsions dans l'image.

Selon un autre mode de réalisation illustré sur la figure 6, la source lumineuse 18 est agencée afin de faire passer la lumière dans la première lamelle 28 de façon perpendiculaire à l'axe optique Z de façon à éclairer l'échantillon 4 ou les objets 22 depuis l'intérieur de la lamelle.

Selon une autre variante, soit une des lamelles 28, 40 est un polariseur, soit les deux lamelles 28, 40 sont des polariseurs croisés. Dans le premier cas, la source lumineuse 18 émet une lumière polarisée rectilignement. Par exemple, la source lumineuse 18 est un écran à cristaux liquides (LCD). Dans le deuxième cas, la source lumineuse 18 n'émet pas de lumière d'excitation polarisée, et la lumière d'excitation est alors polarisée par le second polariseur 40. Dans les deux cas, la lumière d'excitation est arrêtée par le premier polariseur 28 tandis que la lumière d'émission fluorescente issue de l'échantillon 4 est polarisée et transmise par le premier polariseur 28. L'intensité est moindre dans cette configuration mais le rapport signal sur bruit par rapport à une transmission directe de la lumière d'excitation est augmenté. De préférence, le second polariseur 40 est en contact direct avec le liquide consistant le second milieu 42 pour éviter une dépolarisation de la lumière d'excitation à l'interface air/liquide.

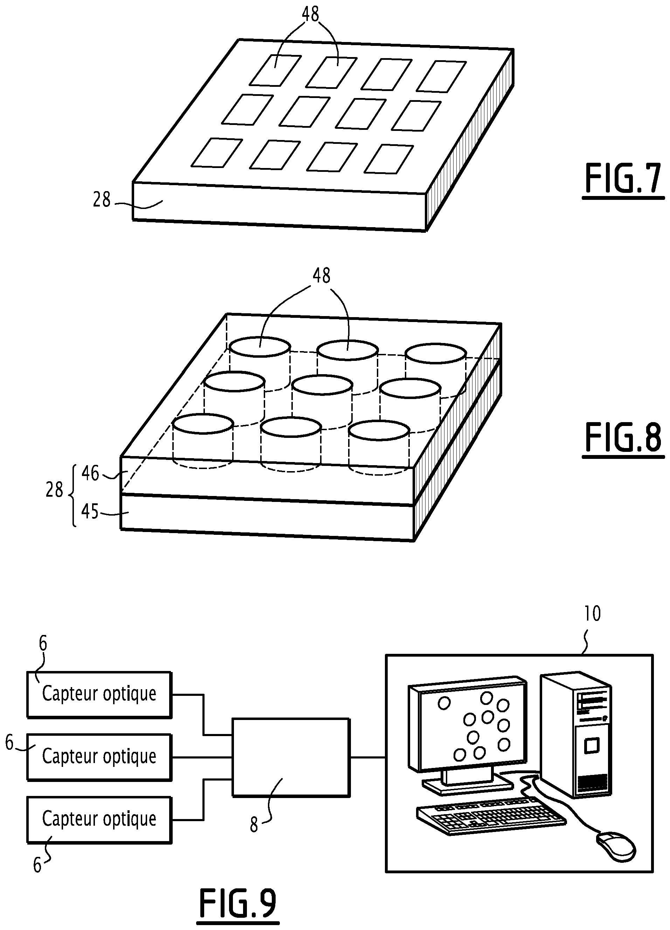

Selon un autre mode de réalisation illustré sur la figure 7, la première lamelle 28 comprend une pluralité de microchambres (ou puits) 48 communicantes ou non communicantes entre elles. Les microchambres 48 sont de préférence arrangées en matrice, par exemple les microchambres 48 sont formées sur ou gravées dans la première lamelle 28. L'épaisseur E intervenant dans le réglage de la distance entre la face 28A de la première lamelle 28 et le sommet des microlentilles 34 est alors la distance mesurée selon l'axe optique Z entre le fond des microchambres 48 et la face 28B de la première lamelle 28.

Selon une variante illustrée sur la figure 8, les microchambres 48 sont formées dans l'intégralité de l'épaisseur E2 de la lamelle amovible 46 précédemment décrite, et l'épaisseur intervenant dans le réglage de la distance H entre la face 28B de la première lamelle 28 et le sommet des microlentilles 34 est alors l'épaisseur E1 de la lame 45.

Selon une variante, la pluralité de microchambres 48 forme une pluralité de zones d'écoulement ou microcanaux de l'échantillon, destinés à faire circuler un fluide, par exemple l'échantillon 4, grâce à des moyens d'actionnement du fluide intégrés au système optique 17 du système d'imagerie 6. De façon connue, les moyens d'actionnement sont des micropompes, des microvalves, etc...

La pluralité de microchambres 48 ou microcanaux est utilisée, par exemple pour réaliser du criblage en parallélisant les observations sur le même système optique 17 du système d'imagerie 6. De façon connue, les observations en parallèle permettent de reproduire les expériences, d'étudier plusieurs types cellulaires et/ou plusieurs densités de cellules, de varier l'organisation spatiale, les concentrations en réactifs, les conditions de culture, etc...

On enregistre alors le signal optique issu de chaque microchambre 48 ou microcanal afin de reconstruire l'image de chaque microchambre 48 ou microcanal.

De manière avantageuse, les images de chaque microchambre 48 ou microcanal correspondent à toute la surface inférieure de chaque puits ou zone d'étude 48 contrairement aux observations réalisées en transmission en microscopie optique classique.

Un autre mode de réalisation destiné à réaliser des études en parallèle est représenté figure 9, le dispositif comprend alors une pluralité de systèmes d'imagerie 6 pilotés en parallèle par un même contrôleur électronique 8. Dans ce cas, le système informatique 10 gère les signaux provenant de chaque système d'imagerie 6 de manière synchronisée ou alternativement.

Selon une variante, le dispositif comprend une pluralité de systèmes d'imagerie 6 pilotés en parallèle par plusieurs contrôleurs électroniques 8. L'utilisation d'un nombre limité de contrôleurs électroniques 8 permet de centraliser la fonction d'interface entre les systèmes d'imagerie selon l'invention et le système informatique 10.

De préférence, les systèmes d'imagerie 6 sont montés sur un même circuit électronique 8 et/ou assemblés dans un boîtier ou sur un râtelier, afin de simplifier la manipulation par l'opérateur et limiter la place prise par le dispositif 2 dans l'incubateur 14.

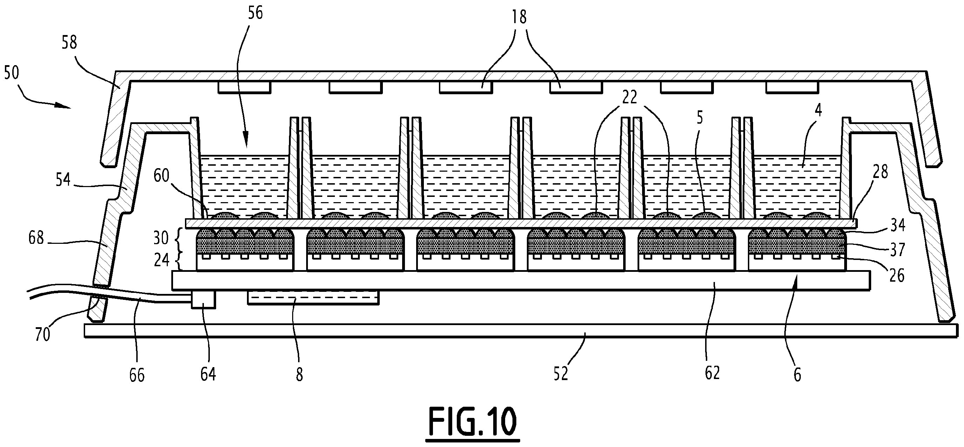

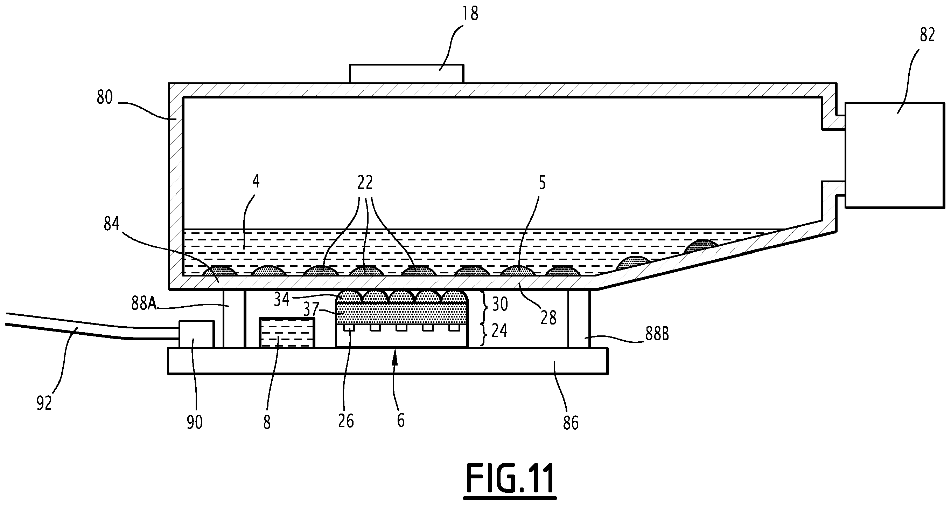

Selon deux modes de réalisation illustrés sur les figures 10 et 11, la première lamelle 28 est respectivement le fond d'une plaque à puits ou la paroi d'un flacon destiné à la culture cellulaire.

De façon avantageuse, les parois des plaques à puits (ou microplaque) et des flacons pour la culture cellulaire sont stériles et ont un traitement de surface favorisant l'adhésion des cellules, par exemple un dépôt de protéines de la matrice extracellulaire ou un plasma oxygène. La stérilité et le traitement de surface améliorent l'attachement, l'étalement et la croissance des cellules sur ces supports, en particulier sur la surface 28A de support des objets 5, de préférence des cellules adhérées 22.

La figure 10 illustre le mode de réalisation d'un dispositif de caractérisation et de détection d'un échantillon selon l'invention dans lequel la première lamelle 28 est le fond d'une plaque à puits ou microplaque 50.

La microplaque 50 comprend une base 52, un support 54 comportant une pluralité de puits 56 et un couvercle 58. Le couvercle est posé sur le support 54, lui-même posé sur la base 52. Le couvercle évite l'évaporation de l'échantillon 4, en particulier du milieu de culture des cellules.

Le support 54 de la microplaque 50 comprend un prolongement inférieur 68. La hauteur totale du support 54, avec le prolongement inférieur 68, est plus grande que la hauteur d'un puits 56. Ainsi, le prolongement inférieur 68 permet de surélever, par exemple d'au moins 1 mm, le fond 60 des puits 56 par rapport à la base 52 de la microplaque. Cet espace disponible entre le fond 60 des puits 56 et la base 52 est destiné au système d'imagerie 6 et au contrôleur électronique 8.

En outre, les quatre coins externes du prolongement inférieur 68 du support 54 de la microplaque 50 sont arrondis avec un rayon de courbure de 3,18 ± 1,6 mm afin que des robots de manipulation de microplaques 50 puissent saisir la microplaque 50 par les coins.

Le dispositif comprend en outre une pluralité de système d'imagerie 6 selon l'invention afin de réaliser des études en parallèle des échantillons contenus dans les différents puits 56.

Dans ce mode de réalisation, la première lamelle 28 de chaque système d'imagerie 6 est commune à tous les systèmes d'imagerie placés sous le support 54 de la microplaque. La face 28A de la première lamelle 28 destinée à supporter les objets 5, de préférence des cellules adhérées 22, forme les fonds 60 de l'ensemble des puits 56 de la microplaque 50. Les fonds 60 sont de préférence plats et transparents au rayonnement lumineux transmis par les objets 5 de l'échantillon 4.

Pour cela, la première lamelle 28 est en verre ou en plastique et collée sous les puits 56 par une colle biocompatible, telle qu'une colle USP class 6 utilisée dans les dispositifs biomédicaux, afin de former le fond 60 des puits 56.

Des marques de métrologie sont déposées ou gravées sur la première lamelle 28 constituant le fond 60 des puits 56, de préférence sur la face inférieure 28B en regard des microlentilles. Les marques de métrologie sont préférentiellement des croix de 30 μm avec une épaisseur de trait de 4 μm, espacées tous les millimètres. Ces marques apparaissent sur les images produites. Les marques de métrologie fournissent une échelle de référence pour mesurer la dimension des cellules et facilitent le recadrage des images lors d'opérations de traitement d'images à l'aide du système informatique 10.

Les parois de la microplaque 50 (base 52, support 54, parois des puits 56 et couvercle 58) autres que le fond des puits sont en polystyrène ou en polypropylène.

En outre, tout ou partie de ces parois sont transparentes au rayonnement lumineux transmis par les objets 5 de l'échantillon 4. Dans ce cas, la microplaque 50 et les systèmes d'imagerie 6 assemblés sous la microplaque 50 sont placés dans une chambre noire pour éviter les perturbations lumineuses externes.

Selon une variante, tout ou partie de ces parois sont opaques de couleur noire ou blanche. De façon connue, les microplaques dont les parois sont opaques et blanches sont utilisées plus particulièrement pour des expériences de luminescence.

De manière préférentielle, les parois des microplaques 50 sont opaques et de couleur noire afin d'éviter les perturbations lumineuses externes, à l'exception de la première lamelle 28. Cette première lamelle 28 est transparente au rayonnement lumineux transmis par les objets 5 de l'échantillon, par exemple en verre ou en plastique.

Lorsque les parois sont opaques et de couleur noire, il n'est pas nécessaire de placer la microplaque 50 recouverte de son couvercle dans une chambre noire pour l'imagerie des objets 5. En effet, les parois de la microplaque 50 avec le couvercle 58 agissent comme une chambre noire. De telles microplaques 50 sont particulièrement appropriées pour des expériences de fluorescence.

Si le couvercle est totalement noir, la ou les sources lumineuses 18 sont fixées sur le couvercle 58 en regard des échantillons contenus dans les puits 56 de la microplaque 50.

Dans le mode de réalisation représenté sur la figure 10, chaque système d'imagerie 6 comprend une source lumineuse 18 qui est donc associée à un seul puits afin d'illuminer l'échantillon à l'intérieur de celui-ci.

Par exemple, la source lumineuse 18 est une feuille électroluminescente ou une LED plate telle qu'une XLamp commercialisée par Cree (Durham, Etats-Unis).

La ou les sources lumineuses 18 peuvent être également placées sur la face extérieure si le couvercle est transparent, ou s'il est opaque et noir avec des fenêtres transparentes au-dessus de chaque puits 56, de manière à illuminer l'intérieur de chaque puits 56.

D'une manière générale, des sources lumineuses 18 émettant peu de chaleur sont sélectionnées pour limiter l'échauffement et l'évaporation de l'échantillon 4.

Une matrice 24 de photocapteurs 26 surmontée d'une matrice 30 d'éléments optiques est positionnée sous chaque puits 56 d'une microplaque 50 afin de visualiser les objets 5 disposés sur le fond 60 de chaque puits 56 de la microplaque 50, en particulier afin de pouvoir réaliser une image de l'échantillon 4, de préférence cellulaire, dans chaque puits 56. L'épaisseur E est alors l'épaisseur de la première lamelle 28, comme dans le mode de réalisation représenté sur la figure 4.

Ainsi, plusieurs expériences sont réalisées et visualisées simultanément dans les différents puits 56 de la microplaque 50.

Chaque système d'imagerie 6 est assemblé sur une carte électronique 62 ou circuit imprimé permettant de réaliser l'interface entre les systèmes d'imagerie et le ou les contrôleurs électroniques 8 du dispositif. La carte électronique 62 est commune à une pluralité de systèmes d'imagerie et à un ou une pluralité de contrôleurs électroniques 8.

Dans une configuration de ce mode de réalisation représentée sur la figure 10, les systèmes d'imagerie sont reliés à un unique contrôleur électronique 8 par la carte électronique 62, afin de centraliser la fonction d'interface entre les systèmes d'imagerie selon l'invention et le système informatique 10.

Les pistes électriques reliant les différents composants électroniques de la carte électronique 62 sont préférentiellement enterrées dans le circuit imprimé ou recouvertes d'un vernis protecteur afin de les protéger de l'atmosphère humide.

La carte électronique 62 est connectée au système informatique 10 via un port électronique 64, connecté sur la carte 62, et un câble 66, relié au port d'une part et au système informatique 10 d'autre part. Une ouverture 70 est percée dans le prolongement inférieur 68 de la microplaque 50 pour le passage du câble 66 reliant le port de connexion 64 et le système informatique 10.

Par exemple, les systèmes d'imagerie 6 sont connectés sur une face de la carte électronique 62 en regard des puits, le port 64 et le contrôleur électronique 8 étant sur la face opposée.

Selon une autre configuration, chaque système d'imagerie 6 est connecté/assemblé sur une carte électronique fille reliée par un connecteur à une carte électronique mère sur laquelle le contrôleur électronique 8 et le port de connexion 64 sont assemblés.

La ou les cartes électroniques 62 sont solidaires de la microplaque 50 grâce à des moyens de fixations mécaniques et de points d'appui disposés entre la microplaque 50 et la ou les cartes électroniques 62.

De façon connue, pour assurer un débit élevé de transmission des images vers le système informatique 10, le port 64 de connexion est un port USB2, FireWire, Ethernet Gigabit ou CamLink.

Selon une autre variante, les communications bidirectionnelles entre le système informatique 10 et la carte électronique 62 sont réalisées par des émetteurs et des récepteurs, et l'alimentation du ou des systèmes d'imagerie 6 est réalisée par une pile ou une batterie à proximité du dispositif.

Les dimensions de la matrice 24 de photocapteurs 26 permettent de couvrir la totalité ou une partie seulement du fond 60 du puits 56 en regard duquel elle est disposée. En outre, les dimensions de la matrice 30 d'éléments optiques sont sensiblement identiques à celles de la matrice 24 de photocapteurs 26 en regard de laquelle elle est disposée.

Par exemple, chaque matrice 24 de photocapteurs 26 a une surface de 3,6 mm x 2,7 mm, placée sous un puits circulaire de 9 mm de diamètre.

Dans un autre exemple, la matrice 24 de photodétecteurs 26 a une surface de 6,4 mm x 4,6 mm placée sous un puits de 18 mm de diamètre.

Pour chaque système d'imagerie 6, le centre des matrices 24 et 30 et celui d'un puits 56 sont alignés.

L'utilisation d'une matrice 24 de photocapteurs 26 et d'une matrice 30 d'éléments optiques de dimensions inférieures au diamètre du puits permet d'éviter d'imager les cellules situées sur le bord du puits dont le comportement est souvent considéré comme non représentatif de la population cellulaire.

De façon connue, les dimensions de la microplaque 50 vérifient les principales spécifications ANSI/SBS 1-2004 à 4-2004 préconisées par la Société pour le Criblage Biomoléculaire (SBS pour « *Society for Biomolecular Screening* » en anglais). Plus spécifiquement, les microplaques possèdent une longueur de 127,76 ± 0,5 mm et une largeur de 85,48 ± 0,5 mm.

Les microplaques 50 possèdent un réseau de 2 x 3, 3 x 4, 4 x 6, 6 x 8, 8 x 12, 16 x 24 ou 32 x 48 puits.

La hauteur totale du support 54 de la microplaque 50 équipée des systèmes d'imagerie 6 est comprise entre 14,35 mm et 35,00 mm, c'est-à-dire la distance, mesurée suivant l'axe optique Z des photocapteurs 26, entre l'extrémité des puits 56 opposée à l'extrémité en contact avec la première lamelle 28 et l'extrémité du prolongement 68 en contact avec la base 52.

La première lamelle 28 est par exemple une plaque de verre de 175 μm ou 210 μm d'épaisseur ou un film transparent de polystyrène de 125 μm d'épaisseur.

Les microplaques 50 intégrées avec les systèmes d'imagerie peuvent être employées entre autres pour des études de prolifération cellulaire, de toxicologie, d'analyses morphologiques, de motilité, de chimiotactisme, d'infection virale, de cancérologie, de criblages pharmaceutiques ou de recherche de molécules influant sur le comportement cellulaire.

La carte électronique 62 a une longueur de 115 mm et une largeur de 75 mm de manière à s'insérer à l'intérieur du prolongement inférieur 68 du support 54 de la microplaque 50. Ainsi, aucun composant de la carte électronique ne touche la base 52 de la microplaque 50. En outre, cette base 52 protège la carte électronique 62 de l'humidité dans l'incubateur 14.

Des systèmes d'imagerie selon l'invention peuvent également être disposés contre la paroi externe d'un ou de plusieurs flacons afin de caractériser les objets disposés sur la paroi interne du flacon ou de plusieurs flacons. L'épaisseur E est alors l'épaisseur de la paroi du flacon.

En regard de la figure 11, un flacon 80 de culture cellulaire est équipé d'un système d'imagerie 6 selon l'invention afin de suivre la prolifération des cellules dans le flacon de culture.

De façon connue, le flacon de culture 80 est une bouteille à parois plates en polystyrène ou en polypropylène qui ont préférentiellement un traitement de surface favorisant l'adhésion cellulaire.

De façon préférentielle, le système d'imagerie 6 est placé sous le flacon 80 de culture de façon à imager les objets, de préférence des cellules 22 adhérées sur la paroi inférieure 84 du flacon 80, la paroi inférieure 84 étant la paroi en regard du système d'imagerie 6. Elle forme la première lamelle 28 du système d'imagerie 6. Ainsi, la paroi inférieure 84 ou seulement une partie a une épaisseur E adaptée pour réaliser une imagerie cellulaire selon l'invention.

Selon une autre variante, le flacon de culture est en polystyrène, à l'exception de la totalité ou d'une partie de la paroi inférieure 84 qui est une lamelle ou une plaque en verre ou en plastique d'épaisseur E. Ladite lamelle, ou plaque en verre ou en plastique, est collée aux parois en polystyrène du flacon de culture à l'aide d'une colle biocompatible telle qu'une colle USP class 6.

Comme dans le mode de réalisation représenté sur la figure 10, le système d'imagerie 6 est connecté au contrôleur électronique 8 par l'intermédiaire d'une carte électronique 86. Un port électronique 90 relié à un câble 92 est assemblé à la carte électronique 86 afin de relier le contrôleur électronique 8 au système informatique 10 du dispositif.

En outre, la carte électronique 86 est solidaire de la paroi inférieure 84 du flacon 80 grâce à des moyens 88A, 88B de fixations mécaniques et de points d'appui disposés entre la paroi inférieure et la carte électronique.

De plus, la source lumineuse 18 est fixée sur la paroi supérieure du flacon 80. En outre, le flacon comprend un bouchon 82 pour éviter l'évaporation de l'échantillon 4, par exemple du milieu de culture cellulaire.

Il est possible d'empiler verticalement les flacons 80 de culture, les systèmes d'imagerie 6 et les sources lumineuses 18 pour un gain de place à l'intérieur de l'incubateur 14. Une source lumineuse très plate telle qu'une feuille électroluminescente est alors choisie pour illuminer l'échantillon du flacon de culture, ce qui facilite l'empilement des flacons et des systèmes d'imagerie. Si un autre type de source lumineuse est choisi, un espace pour la source lumineuse est réservé au-dessus de chaque flacon de culture à l'aide de moyens de fixation et de points d'appui.

Les images collectées par le système informatique 10 peuvent être consultées à distance grâce à un serveur informatique. L'utilisateur accédant à distance aux images des cellules peut décider de changer ou non le milieu de culture des cellules ou repiquer les cellules dans un nouveau flacon de culture.

Des microbilles de taille calibrée peuvent être introduites dans l'échantillon afin de mesurer la température du milieu à partir des images produites. En effet, de façon connue, les microbilles apparaissent sur les images et le déplacement d'une microbille entre deux images successives dépend du mouvement brownien, c'est-à-dire de la température. Ainsi, la mesure du déplacement d'une microbille entre deux images successives permet de déduire la température en utilisant l'équation de Stokes-Einstein :

$$T = \frac{3\pi r \eta <d^2>}{k\,t}$$

avec $r$ le rayon de la microbille, $\eta$ la viscosité du milieu, $<d^2>$ la distance au carré parcourue par la microbille pendant le temps $t$, et $k$ la constante de Boltzmann ($k = 1{,}3806504.10^{-23}\ J\,.K^{-1}$).

La mesure précise de la position des billes sur une image est réalisée en utilisant comme références les marques de métrologie déposées ou gravées sur la lamelle 28 comme indiqué précédemment, et ce quelque soit le mode de réalisations du système d'imagerie (microplaque, flacon de culture...).

Le système d'imagerie et le dispositif associé sont destinés à détecter et caractériser optiquement des objets, en particulier des cellules.

Les microlentilles focalisent la lumière issue des zones de l'échantillon dans les zones sensibles correspondantes du silicium afin de collecter le maximum de lumière. L'intensité lumineuse est intégrée par chaque photocapteur. La matrice de microlentilles modifie le découpage (discrétisation) de l'espace réalisé par la matrice de photocapteurs.

De plus, la première lamelle 28 engendre des effets de diffraction et d'interférence qui favorisent la visualisation d'objets à faible contraste tels que des cellules adhérées à la surface de la première lamelle, en particulier le contour de ces objets.

Le champ de vision du système d'imagerie 6 correspond aux dimensions de la matrice 24 de photocapteurs 26. A titre d'exemple, le champ de vision est élargi par rapport à un microscope classique sur un rectangle de plusieurs mm$^2$ et jusqu'à plusieurs cm$^2$ de surface. Actuellement les images obtenues correspondent à un grossissement x4 en microscopie classique mais sur un champ plus grand d'environ 3,5 x 4,5 mm$^2$ par rapport à un objectif d'ouverture numérique classique. Le grandissement des images diffère en fonction de la distance entre la face 28A, support de l'échantillon, de la première lamelle 28 et le sommet des microlentilles 34. En effet, si l'échantillon 4 ou les objets 22 sont éloignés de la matrice 24 de photocapteurs 26, l'image de l'échantillon ou des objets est étalés sur un nombre croissant de photocapteurs 26.

La résolution dépend de la taille des pixels, cependant elle dépend également de la focale des microlentilles, de la distance entre l'échantillon 4 ou les objets 22 et la matrice de photocapteurs, et de l'indice de réfraction n du milieu 36.

Le dispositif a des dimensions réduites, de préférence celle d'un cube de 10 cm de coté et est facilement installé dans un incubateur.

Les dimensions réduites du dispositif présentent l'avantage de la grande portabilité du dispositif, en envisageant des applications au lit du patient, tel que le comptage de cellules du sang, ou de pouvoir être placé directement dans un incubateur en ajoutant seulement la présence d'un ordinateur dans la pièce de culture cellulaire.

L'invention a été décrite dans le cadre d'applications biologiques avec des échantillons comprenant des cellules. Elle s'applique à tout domaine avec des cellules, par exemple pour visualiser des bactéries, des levures, des champignons, des pollens, des algues.

## Revendications

1. Système d'imagerie d'un échantillon (4) comprenant une matrice (24) de photocapteurs (26), une première lamelle (28) disposée en regard de la matrice (24) de photocapteurs définissant une face (28A) de support de l'échantillon, et un ensemble (30) d'éléments optiques, disposé entre la matrice (24) de photocapteurs et la première lamelle (28), l'ensemble (30) d'éléments optiques comportant une matrice (32) de microlentilles (34), une microlentille (34) étant située au-dessus de chaque photocapteur (26) de la matrice (24) de photocapteurs et **caractérisé en ce que** :

   - l'ensemble (30) d'éléments optiques comprend un milieu optique (36) disposé entre la matrice (32) de microlentilles et la première lamelle (28), le milieu étant de l'air, du gel ou du liquide et l'indice de réfraction du milieu optique (36) étant sensiblement compris entre 1 et l'indice de réfraction des microlentilles (34),
   - la distance mesurée selon l'axe optique (Z) des photocapteurs (26) entre la face de support de l'échantillon (28A) et le sommet des microlentilles (34) est sensiblement comprise entre 100 $\mu$m et 1500 $\mu$m,
   - le système d'imagerie comporte l'échantillon (4) comprenant des cellules biologiques (5),
   - les photocapteurs (26) associés aux microlentilles (34) sont agencés pour que l'image d'une cellule biologique (5) soit couverte par au moins deux photocapteurs (26) adjacents dans une première direction et deux autres photocapteurs (26) adjacents dans une seconde direction perpendiculaire à la première direction,
   - la focale de chaque microlentille (34) est inférieure à 25 $\mu$m, et
   - l'axe optique de chaque microlentille (34) est sensiblement confondu avec l'axe optique d'un photocapteur (26).

2. Système d'imagerie selon la revendication 1, dans lequel la distance mesurée selon l'axe optique (Z) des photocapteurs (26) entre la face de support de l'échantillon (28A) et le sommet des microlentilles (34) est inférieure ou égale à -150$\times$F$\times$n+400$\times$F, où F est la focale des microlentilles (34) mesurée dans l'air et n l'indice de réfraction du milieu optique (36) et en ce que l'indice de réfraction du milieu optique (36) est sensiblement compris entre 1 et 1,64.

3. Système d'imagerie selon la revendication 1 ou 2, comprenant une source lumineuse (18) destinée à éclairer l'échantillon (4), la source lumineuse (18) étant disposée au-dessus de l'échantillon afin de réaliser une image en transmission.

4. Système d'imagerie selon la revendication 1 ou 2, comprenant une source lumineuse (18) destinée à éclairer l'échantillon (4), la source lumineuse (18) étant disposée afin d'éclairer dans la première lamelle (28) du système d'imagerie (6) de façon perpendiculaire à l'axe optique du système d'imagerie (6).

5. Système d'imagerie selon la revendication 3 ou 4, dans lequel la source lumineuse (18) est apte à éclairer avec une longueur d'onde comprise entre 200 nm et 800 nm.

6. Système d'imagerie selon la revendication 5, dans lequel la source lumineuse (18) est une lumière blanche.

7. Système d'imagerie selon l'une quelconque des revendications 1 à 6, dans lequel le milieu optique (36) est un liquide.

8. Système d'imagerie selon la revendication 7, comprenant une cuvette (38) contenant le liquide et l'échantillon.

9. Système d'imagerie selon l'une quelconque des revendications 1 à 8, comprenant une seconde lamelle (40) disposée telle que l'échantillon (4) est situé entre la première lamelle (28) et la seconde lamelle (40).

10. Système d'imagerie selon l'une quelconque des revendications 1 à 9, dans lequel la face (28A) de support de l'échantillon (4) de la première lamelle (28) est disposée en regard de la matrice de photocapteurs.

11. Système d'imagerie selon l'une quelconque des revendications 1 à 10, dans lequel la première lamelle (28) comporte une lame (45) et une lamelle amovible (46) accolée à ladite lame (45) et définissant la face (28A) de support de l'échantillon (4).

12. Système d'imagerie selon l'une quelconque des revendications 1 à 11, comprenant des moyens de réglage (44) de la distance, mesurée selon l'axe optique (Z) des photocapteurs (26), entre la face de support de l'échantillon (28A) et le sommet des microlentilles (34).

**13.** Système d'imagerie selon l'une quelconque des revendications 1 à 12, dans lequel la première lamelle (28) comprend une pluralité de microchambres (48) ou microcanaux.

**14.** Système d'imagerie selon l'une quelconque des revendications 1 à 13, dans lequel les photocapteurs (26) sont aptes à détecter une lumière émise entre 200 nm et 800 nm par bioluminescence, chimiluminescence ou fluorescence.

**15.** Système d'imagerie selon l'une quelconque des revendications 1 à 14, dans lequel le diamètre d'ouverture de chaque microlentille (34) est sensiblement compris entre 0,7 $\mu$m et 10 $\mu$m.

**16.** Dispositif (2) de caractérisation et de détection d'un échantillon (4) **caractérisé en ce qu'**il comprend un système d'imagerie (6) selon l'une quelconque des revendications 1 à 15, un contrôleur électronique (8) et un système informatique (10) destiné à commander le système d'imagerie (6).

**17.** Dispositif selon la revendication 16, comprenant au moins deux systèmes d'imagerie (6) selon l'une quelconque des revendications 1 à 15, pilotés en parallèle par le contrôleur électronique (8).

**18.** Dispositif selon la revendication 17, dans lequel la première lamelle (28) est commune à au moins deux systèmes d'imagerie (6) adjacents.

**19.** Dispositif selon l'une quelconque des revendications 16 à 18, comprenant une chambre d'incubation (14) dans laquelle le système d'imagerie (6) et l'échantillon (4) sont placés.


**Patentansprüche**

**1.** System zum Abbilden einer Probe (4), welches aufweist eine Matrix (24) an Photosensoren (26), eine erste Lamelle (28), welche gegenüber von der Matrix (24) an Photosensoren angeordnet ist und welche eine Fläche (28A) zum Tragen der Probe definiert, und eine Gruppe (30) von optischen Elementen, welche zwischen der Matrix (24) an Photosensoren und der ersten Lamelle (28) angeordnet ist, wobei die Gruppe (30) von optischen Elementen eine Matrix (32) an Mikrolinsen (34) aufweist, wobei eine Mikrolinse (34) oberhalb von jedem Photosensor (26) der Matrix (24) an Photosensoren angeordnet ist, und **dadurch gekennzeichnet, dass**

- die Gruppe (30) von optischen Elementen ein optisches Medium (36) aufweist, welches zwischen der Matrix (32) an Mikrolinsen und der ersten Lamelle (28) angeordnet ist, wobei das Medium Luft, Gel oder Flüssigkeit ist und der Brechungsindex des optischen Mediums (36) im Wesentlichen zwischen 1 und dem Brechungsindex der Mikrolinsen (34) liegt,
- die entlang der optischen Achse (Z) der Photosensoren (26) gemessene Entfernung zwischen der Fläche zum Tragen der Probe (28A) und dem Scheitelpunkt der Mikrolinsen (34) im Wesentlichen zwischen 100 $\mu$m und 1500 $\mu$m liegt,
- das System zum Abbilden die Probe (4) aufweist, welche biologische Zellen (5) aufweist,
- die mit den Mikrolinsen (34) assoziierten Photosensoren (26) angeordnet sind, damit das Bild einer biologischen Zelle (5) von mindestens zwei Photosensoren (26), welche in einer ersten Richtung benachbart sind, und zwei weiteren Fotosensoren (26), welche in einer zweiten Richtung, welche zur ersten Richtung senkrecht ist, benachbart sind, abgedeckt wird,
- die Brennweite jeder Mikrolinse (34) kleiner als 25 $\mu$m ist, und
- die optische Achse jeder Mikrolinse (34) im Wesentlichen mit der optischen Achse eines Photosensors (26) zusammenfällt.

**2.** System zum Abbilden gemäß Anspruch 1, wobei die entlang der optischen Achse (Z) der Photosensoren (26) gemessene Entfernung zwischen der Fläche zum Tragen der Probe (28A) und dem Scheitelpunkt der Mikrolinsen (34) kleiner oder gleich - 150 x F x n + 400 x F ist, wobei F die in der Luft gemessene Brennweite der Mikrolinsen (34) und n der Brechungsindex des optischen Mediums (36) ist, und dadurch, dass der Brechungsindex des optischen Mediums (36) im Wesentlichen zwischen 1 und 1,64 liegt.

**3.** System zum Abbilden gemäß Anspruch 1 oder 2, welches eine Lichtquelle (18) aufweist, welche dazu bestimmt ist, die Probe (4) zu beleuchten, wobei die Lichtquelle (18) oberhalb von der Probe angeordnet ist, um ein Bild in Transmission zu erzeugen.

**4.** System zum Abbilden gemäß Anspruch 1 oder 2, welches eine Lichtquelle (18) aufweist, welche dazu bestimmt ist, die Probe (4) zu beleuchten, wobei die Lichtquelle (18) angeordnet ist, um in die erste Lamelle (28) des Systems zum Abbilden (6) senkrecht zur optischen Achse des Systems zum Abbilden (6) zu leuchten.

**5.** System zum Abbilden gemäß Anspruch 3 oder 4, wobei die Lichtquelle (18) geeignet ist, um mit einer Wellenlänge zu beleuchten, welche zwischen 200 nm und 800 nm liegt.

**6.** System zum Abbilden gemäß Anspruch 5, wobei die Lichtquelle (18) ein weißes Licht ist.

7. System zum Abbilden gemäß irgendeinem der Ansprüche 1 bis 6, wobei das optische Medium (36) eine Flüssigkeit ist.

8. System zum Abbilden gemäß Anspruch 7, welches eine Küvette (38) aufweist, welche die Flüssigkeit und die Probe enthält.

9. System zum Abbilden gemäß irgendeinem der Ansprüche 1 bis 8, welches eine zweite Lamelle (40) aufweist, welche derart angeordnet ist, dass die Probe (4) zwischen der ersten Lamelle (28) und der zweiten Lamelle (40) angeordnet ist.

10. System zum Abbilden gemäß irgendeinem der Ansprüche 1 bis 9, wobei die Fläche (28A) zum Tragen der Probe (4) der ersten Lamelle (28) gegenüber von der Matrix an Photosensoren angeordnet ist.

11. System zum Abbilden gemäß irgendeinem der Ansprüche 1 bis 10, wobei die erste Lamelle (28) aufweist einen Träger (45) und eine lösbare Lamelle (46), welche mit dem besagten Träger (45) verbunden ist und die Fläche (28A) zum Tragen der Probe (4) definiert.

12. System zum Abbilden gemäß irgendeinem der Ansprüche 1 bis 11, welches aufweist Mittel zum Einstellen (44) der entlang der optischen Achse (Z) der Photosensoren (26) gemessenen Entfernung zwischen der Fläche zum Tragen der Probe (28A) und dem Scheitelpunkt der Mikrolinsen (34).

13. System zum Abbilden gemäß irgendeinem der Ansprüche 1 bis 12, wobei die erste Lamelle (28) eine Mehrzahl von Mikrokammern (48) oder Mikrokanälen aufweist.

14. System zum Abbilden gemäß irgendeinem der Ansprüche 1 bis 13, wobei die Photosensoren (26) imstande sind, ein Licht zu detektieren, welches zwischen 200 nm und 800 nm mittels Biolumineszenz, Chemilumineszenz oder Fluoreszenz emittiert wird.

15. System zum Abbilden gemäß irgendeinem der Ansprüche 1 bis 14, wobei der Öffnungsdurchmesser jeder Mikrolinse (34) im Wesentlichen zwischen 0,7 $\mu$m und 10 $\mu$m liegt.

16. Vorrichtung (2) zum Charakterisieren und zum Detektieren einer Probe (4), **dadurch gekennzeichnet, dass** es aufweist ein System zum Abbilden (6) gemäß irgendeinem der Ansprüche 1 bis 15, eine elektronische Steuereinheit (8) und ein Computersystem (10), welches dazu bestimmt ist, das System zum Abbilden (6) zu steuern.

17. Vorrichtung gemäß Anspruch 16, welche zumindest zwei Systeme zum Abbilden (6) gemäß irgendeinem der Ansprüche 1 bis 15 aufweist, welche parallel mittels der elektronischen Steuereinheit (8) gesteuert werden.

18. Vorrichtung gemäß Anspruch 17, wobei die erste Lamelle (28) zumindest zwei benachbarten Systemen zum Abbilden (6) gemeinsam ist.

19. Vorrichtung gemäß irgendeinem der Ansprüche 16 bis 18, welche eine Inkubationskammer (14) aufweist, in welcher das System zum Abbilden (6) und die Probe (4) angeordnet sind.

**Claims**

1. A system for imaging a sample (4) comprising a matrix (24) of photosensors (26), a first lamina (28) disposed opposite the matrix (24) of photosensors defining a face (28A) for supporting the sample, and a set (30) of optical elements disposed between the matrix (24) of photosensors and the first lamina (28), the set (30) of optical elements comprising a matrix (32) of microlenses (34), a microlens (34) being situated above each photosensor (26) of the matrix (24) of photosensors and **characterized in that:**

   - the set (30) of optical elements comprises an optical medium (36) disposed between the matrix (32) of microlenses and the first lamina (28), the medium being air, gel or liquid, the refractive index of the optical medium (36) being substantially between 1 and the refractive index of the microlenses (34),
   - the distance measured along the optical axis (Z) of the photosensors (26) between the face (28A) supporting the sample and the apex of the microlenses (34) is substantially between 100 $\mu$m and 1500 $\mu$m,
   - the system for imaging comprises the sample (4) comprising cells (5),
   - the photosensors (26) associated with the microlenses (34) are arranged so that an imgage cell (5) is covered by at least two adjacent photosensors in a first direction and two other adjacent photosensors in a second direction perpendicular to the first direction,
   - the focal of each microlens (24) is inferior to 25 $\mu$m, and
   - the optical axis of each microlens (24) is substantially coaxial with the optical axis of a photosensor (26).

2. The imaging system according to claim 1, wherein the distance measured along the optical axis (Z) of the photosensors (26) between the support face (28A) of the sample and the apex of the microlenses (34) is smaller than or equal to -150×F×n+400×F, where F is the focal length of the microlenses (34) measured in the air and n the refractive index of the optical medium (36) and in that the refractive index of the optical medium (36) is substantially between 1 and 1.64

3. The imaging system according to claim 1 or 2, comprising a light source (18) intended to light the sample (4), the light source (18) being disposed above the sample so as to produce a transmission image.

4. The imaging system according to claim 1 or 2, comprising a light source (18) intended to light the sample (4), the light source (18) being disposed so as to light in the first lamina (28) of the imaging system (6) perpendicularly to the optical axis of the imaging system (6).

5. The imaging system according to claim 3 to 4, wherein the light source (18) is able to light with a wavelength comprised between 200 nm and 800 nm.

6. The imaging system according to claim 5, wherein the light source (18) is a white light.

7. The imaging system according to any one of claims 1 to 6, wherein the optical medium (36) is a liquid.

8. The imaging system according to claim 7, comprising a cuvette (38) containing the liquid and the sample.

9. The imaging system according to any one of claims 1 to 8, comprising a second lamina (40) disposed so that the sample (4) is situated between the first lamina (28) and the second lamina (40).

10. The imaging system according to any one of claims 1 to 9, **characterized in that** the support face (28A) of the sample (4) of the first lamina (28) is disposed opposite the matrix of photosensors.

11. The imaging system according to any one of claims 1 to 10, wherein the first lamina (28) includes a slide (45) and a removable lamina (46) alongside the slide (45) and defining the support face (28A) of the sample (4).

12. The imaging system according to any one of claims 1 to 11, comprising means (44) for adjusting the distance, measured along the optical axis (Z) of the photosensors (26), between the support surface (28A) of the sample and the apex of the microlenses (34).

13. The imaging system according to any one of claims 1 to 12, wherein the first lamina (28) comprises a plurality of microchambers (48) or microchannels.

14. The imaging system according to any one of claims 1 to 13, wherein the photosensors can detect a light emitted between 200 nm and 800 nm by bioluminescence, chemiluminescence or fluorescence.

15. The imaging system according to any one of claims 1 and 14, wherein the opening diameter of each microlens is substantially comprised between 0.7 and 10 μm.

16. A device (2) for detecting and characterizing a sample (4) **characterized in that** it comprises an imaging system (6) according to any one of claims 1 to 15, an electronic controller (8) and a computer system (10) intended to control the imaging system (6).

17. The device according to claim 16, comprising at least two imaging systems (6) according to any one of claims 1 to 15, steered in parallel by the electronic controller (8).

18. The device according to claim 17, wherein the first lamina (28) is shared by at least two adjacent imaging systems (6).

19. The device according to any one of claims 16 to 18, comprising an incubation chamber (14) in which the imaging system (6) and the sample (4) are placed.

## FIG.1

## FIG.2

## FIG.3

## FIG.4

## FIG.5

## FIG.6

FIG.7

FIG.8

FIG.9

Capteur optique

Capteur optique

Capteur optique

FIG.10

FIG.11

EP 2 438 428 B1

**EP 2 438 428 B1**

## RÉFÉRENCES CITÉES DANS LA DESCRIPTION

**Documents brevets cités dans la description**

- US 2001021534 A1